Europäisches Patentamt

European Patent Office

Office européen des brevets

(19)

(11) EP 0 937 469 A2

(12) **EUROPEAN PATENT APPLICATION**

(43) Date of publication:
25.08.1999 Bulletin 1999/34

(51) Int Cl.$^6$: **A61L 27/00**

(21) Application number: 99103567.6

(22) Date of filing: 24.02.1999

(84) Designated Contracting States:
**AT BE CH CY DE DK ES FI FR GB GR IE IT LI LU MC NL PT SE**
Designated Extension States:
**AL LT LV MK RO SI**

(30) Priority: 24.02.1998 IT TO980138

(71) Applicant: **SORIN BIOMEDICA CARDIO S.p.A.**
**I-13040 Saluggia, Vercelli (IT)**

(72) Inventors:
• **Rinaldi, Stefano**
**43100 Parma (IT)**
• **Della Ciana, Leopoldo**
**48022 Lugo (Ravenna) (IT)**
• **Vassarotto, Cristina**
**10147 Torino (IT)**
• **Castelli, Raffaele**
**20025 Legnano (Milano) (IT)**

(74) Representative: **Gerbino, Angelo et al**
**c/o JACOBACCI & PERANI S.p.A.**
**Corso Regio Parco, 27**
**10152 Torino (IT)**

(54) **A coated vascular prosthesis and a method for its production**

(57)    The vascular prosthesis has a body made from biostable and biocompatible polymeric material, and at least one surface portion thereof is coated with an impermeable layer based on a synthetic, biodegradable and biocompatible polymer having a coefficient of elasticity less than 10 MPa. Preferably, this polymer is a poly-(lactide-caprolactone) copolymer having an intrinsic viscosity [η], measured in chloroform at 25°C, of less than 4 dl/g. The impermeable layer can be applied using coating or rolling techniques.

FIG. 1

CL-DLLA COPOLYMERS: GLASS TRANSITION AND MELTING TEMPERATURES

| %PCL | Tg | Tm | %PDLLA |
|------|------|------|--------|
| 100 | -60 | 59.7 | 0 |
| 80 | -50.9 | 36.5 | 20 |
| 60 | -28 | | 40 |
| 40 | -1 | | 60 |
| 0 | 49 | | 100 |

## Description

The present invention concerns a vascular prosthesis and a method for its production.

Prostheses of this kind are formed, for example, from textiles, meshes or other porous structures woven from PET, PTFE, or other biostable and biocompatible polymers. Porosity is an essential requirement for these prostheses as it assists in the incorporation of the prosthesis in the surrounding tissues and/or the newly formed tissues (the pseudo-neointima) following implantation. However, this porosity, if not limited in some way, would disadvantageously allow an undesirable movement of blood through the walls of the prostheses.

In order to avoid this, the prosthesis is treated with the patient's blood before implantation, so-called "pre-clotting", in order to render the prosthesis impermeable by means of thrombotic deposits (principally fibrin) of autologous blood.

However, the "pre-clotting" treatment is very delicate, and its success depends upon a number of factors that are not always easily controllable, such as the chemical composition of the patient's blood in which the prosthesis is implanted, the possible presence of drugs, the manner of execution of the procedure and the elapsed time between the "pre-clotting" treatment itself and the implantation of the prosthesis. In any case, there is always a risk of bacterial contamination associated with handling the prosthesis and the blood.

In order to avoid these disadvantages, it has been proposed to impregnate the prosthesis with a substance that makes it impermeable to blood, thus eliminating the need for the "pre-clotting" treatment. This substance also acts as a temporary "scaffold" after implantation, promoting cellular growth, the incorporation of the prosthesis in the surrounding tissues and the "graft healing" process, becoming gradually reabsorbed during the "healing" period and replaced by the host's tissue.

Examples of substances that have been proposed for prosthesis impregnation and which have the aforesaid characteristics include collagen, which is the most common structural protein in mammalian tissues, gelatine, which is the denatured form of collagen, and albumin.

In practice, gelatine and, especially, collagen have been the substances utilised until now for the impregnation of prostheses, while albumin has been little used because of source limitations.

For example, US-A-4 842 575 describes a method for the impregnation of synthetic vascular prostheses using, in particular, a PET such as Dacron (registered trade mark) with an aqueous collagen slurry.

By virtue of the elasticity, resistance and the hydrophilic nature of the constituent fibrils, this substance exhibits "in vivo" structural and coating functions, and has good biocompatibility and absorbability. In fact, when it is extracted from mammalian tissues and subjected to appropriate chemical treatment, it has a low antigenicity (so that it is not recognised as a foreign body by the organism into which it is implanted) and is therefore biodegraded in vivo, releasing sub-products that are metabolised in normal biological exchange mechanisms similar to those that occur during "tissue repair".

For the reasons described above, collagen is widely used as a prosthesis-impregnating substance, although it also has a number of disadvantages.

In fact, collagen is very susceptible to bacterial attack and it is therefore necessary to work under sterile conditions in order to minimise the formation of bacteria, as this could lead to possible attack by bacterial proteases that could degrade the material, as well as leading to difficulties in sterilising the finished product and the possible pyrogenicity of the prostheses.

It is also difficult to obtain purified collagen having uniform characteristics because of the different ages of the animals from which it is extracted and the complexity of the purification process which is, in any case, very expensive. Therefore, due to the inhomogeneity of the starting product, problems can arise during the impregnation treatment, which is not always perfectly reproducible and repeatable.

Furthermore, there is a risk that the collagen will be extracted in Europe from animal tissues contaminated by slow viruses, for example, BSE.

Finally, collagen can be denatured at temperatures above 40°C. Therefore, the manners in which it is handled and transported must be strictly controlled in order to avoid exceeding this threshold temperature.

The object of the present invention is to overcome the disadvantages described above in relation to the known techniques for making vascular prostheses impermeable.

This object is achieved in the form of a coated vascular prosthesis as defined specifically in the following claims which also refer to the associated methods of production.

The invention is further illustrated in the accompanying drawings, given by way of non-limitative example, in which:

Figure 1 illustrates the variation in the glass transition and melting temperatures of poly-(lactide-caprolactone) (DLLA-CL) copolymers as a function of the percentage of caprolactone;
Figure 2 illustrates the variation in the maximum load of CL-DLLA copolymers as a function of the percentage of caprolactone;
Figure 3 illustrates the variation in the coefficient of elasticity of CL-DLLA copolymers as a function of the percentage of caprolactone;
Figure 4 illustrates the variation in a time degradation constant of CL-DLLA copolymers as a function of the percentage of caprolactone; and
Figure 5 is a SEM photographic representation of a polymer layer according to the invention applied to a prosthesis body made from Dacron (registered trade mark).

Since, in the prostheses of the invention, the impermeable coating layer is made from a synthetic polymer, the risk of possible contamination by pathogenic agents is avoided, as are the problems of reproducibility related to the use of materials of natural origin.

These synthetic polymers are also biodegradable and biocompatible (this also includes the products of degradation) and, as they are preferably hydrophobic and anhydrous, they demonstrate good properties of adhesion to the prosthesis body.

Since these polymers have good elastic properties and do not penetrate deeply into the prosthesis body, the constituent fibres of the prosthesis maintain their original flexibility and softness which means that their suturability and the possibilities for handling the coated prosthesis are more than satisfactory.

According to the invention, the impermeable coating can be applied to the outer surface of the prosthesis, to its inner surface, or to both surfaces using conventional application techniques which are reproducible, controllable and inexpensive. These techniques can, for example, be based on coating operations, possibly performed in several stages, or rolling, possibly at high temperatures, and/or a combination thereof and possibly followed by or alternated with heat treatments.

Furthermore, different techniques can be used to apply the impermeable layer to areas of a single prosthesis having different geometries, for example, the straight portions and any branching areas that may be present.

Advantageously, the impermeable coating of the prosthesis body is between 50 and 300 μm thick.

Polymers that can be used to form the impermeable coating according to the invention are, for example, the poly(lactide-caprolactone) copolymers having an intrinsic viscosity [η], measured in chloroform at 25°C, of less than 4 dl/g, preferably between 0.5 and 4 dl/g, and more preferably equal to 2 dl/g. These values indicate a molecular weight of at least 40,000 Dalton.

Advantageously, these copolymers are obtained from D,L-lactide and ε-caprolactone in a weight ratio of between 40%/ 60% and 60%/40% and, preferably, equal to 50%/50%.

These copolymers are amorphous, having a glass transition temperature of between 0° and -30°C, a coefficient of elasticity less than 10 MPa, and an elastic breaking strain greater than 100%. These properties are such that a coating formed from these copolymers, once applied to a prosthesis body, has optimal characteristics of impermeability, elasticity and speed of degradation, which make it suitable for use on an implanted prosthesis.

This result is surprising when one considers the fact that the homopolymers both of D, L-lactide and ε-caprolactone are rigid and degrade very slowly. However, it must be taken into account that these properties arise from very different characteristics and from certain opposite standpoints.

Poly-caprolactone is, in fact, a polymer having a high degree of crystallinity, with a melting point $T_m$ of approximately +60°C, and a low glass transition temperature $T_g$ (approximately -60°C). The high degree of crystallinity (structural order) further confers rigidity and limits the quantity of water that can be absorbed by the material and the velocity at which it can defuse therein and, consequently, it slows down the hydrolysis responsible for the biodegradation.

On the other hand, poly-D,L-lactide is an amorphous polymer, but has a $T_g$ of +49°C. Therefore, at ambient temperature and at the temperature of use (+37°C), it is at a temperature less than the $T_g$ and, therefore, exists in a rigid "glassy" state with reduced molecular mobility.

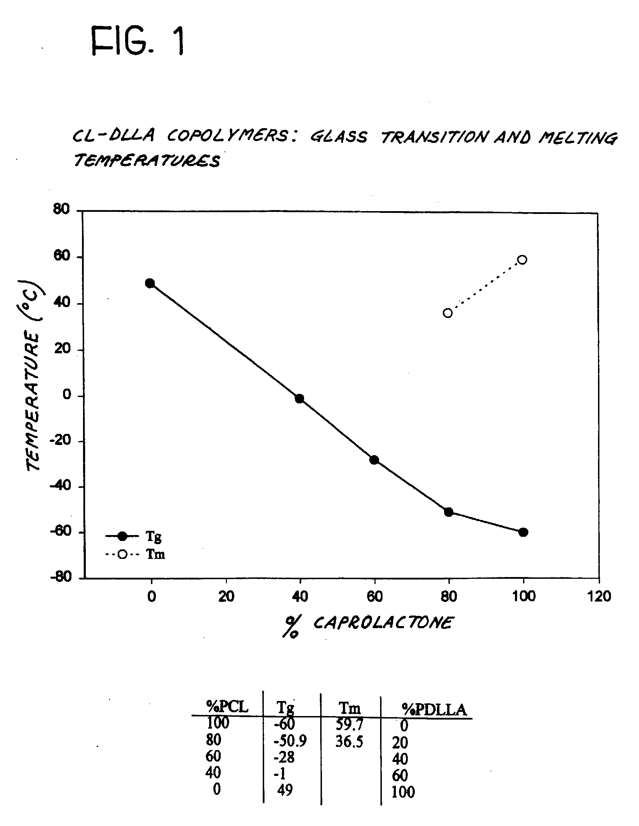

By combining the two monomers in variable percentages to form the copolymer (see Figure 1), it is possible to identify a composition interval (corresponding to a percentage of caprolactone of between approximately 40% and 60%) in which the degree of crystallinity is extremely low or nil and which, at the same time, exhibits a $T_g$ that is still significantly less than ambient temperature. Because of this, the resulting copolymer is elastic.

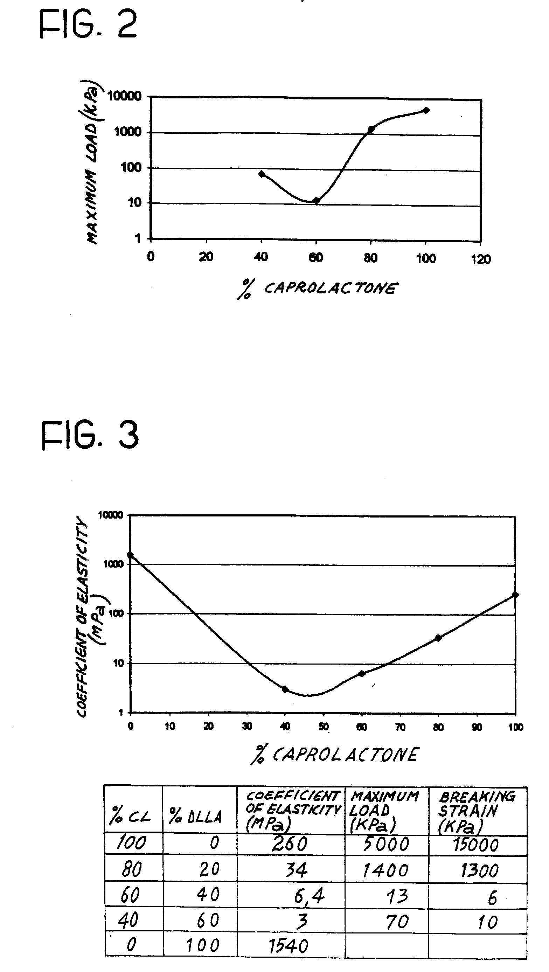

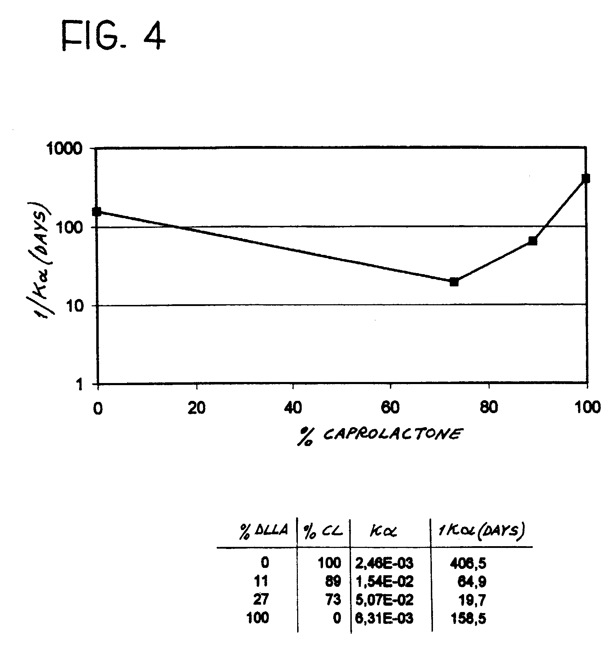

These characteristics are illustrated in Figures 2, 3 and 4, which show - as a function of the variation in composition of CL-DLLA copolymers - the respective variations of the maximum load, the coefficient of elasticity and a time degradation constant, defined as the inverse of the quantity Kxα, in which:

α is the exponent of the Mark-Houwing equation

$$\eta = constant \times M^{\alpha}_{w}$$

which describes the correlation between the intrinsic viscosity and the molecular weight of a polymer ($M_w$); K is the gradient of the straight line which, on a logarithmic scale, describes the decrease of η over time due to the hydrolysis in polymers kept in water or aqueous solution.

Other examples of polymers which can be used according to the invention to form an impermeable coating on a prosthesis are provided by the aliphatic polyesters. Homopolymers or copolymers of polyvalerolactone, ε-decalactone, polyhydroxybutyrate, polyhydroxyvalerate, polydioxanone, can be added as plasticisers.

These plasticisers can be, for example, monomers or oligomers of the same aliphatic polyesters, as well as lecithin, polyvinyl pyrrolidone (PVP), esters of citric acid, esters of trimellitic acid and glycerololigolactides. The same plasticisers can also be added to the ε-CL/DLLA copolymers described above.

Further advantages and characteristics of the present invention will become clear from the following examples of methods for producing coated prostheses, given by way of non-limitative example.

EXAMPLE 1

A copolymer of D,L-lactide and ε-caprolactone is prepared with a weight ratio between the components of 50% / 50%, and an intrinsic viscosity [η], measured in chloroform at 25°C, equal to 2 dl/g, according to methods described, for example, in:

M. Hiljanen-Vainio et al. "Biodegradable lactone copolymers. I. Characterisation and mechanical behaviour of ε-Caprolactone and Lactide Copolymers", J. of Applied Polymer Science (1996), 59; 1281-1288;
X. Zhang et al., "An investigation of the synthesis and thermal stability of poly(D;L-lactide)", J.Polym. SCI. Polym. Chem. (1994), 32: 2965-2970;
D. W. Grijpma et al., "Polimerization temperature effects on the properties of L-lactide and ε-caprolactone copolymers", Polymer Bulletin (1991), 25: 335-341;
M. P. Hiljanen-Vainio et al., "Properties of ε-caprolactone/DL-lactide (ε-CL/DLLA) copolymers with a minor ε-CL content", J. Biomed. Mat. Res. (1997), 34: 39-46.

A 5% solution of the copolymer in acetone, 2-butanone, dimethylacetamide or mixtures thereof is then prepared. The solution is then placed in an evaporator made from an anti-adhesive material such as PTFE, and within a mould in order to obtain, after the evaporation of the solvent, a copolymer sheet having a shape corresponding to that of the mould, for example, rectangular or lozenge shape.

The sheet thus obtained is then put on a slab of polished anti-adhesive material, on which a template of anti-adhesive material is placed, which template reproduces the geometry of the copolymer film intended to be deposited on the body of a prosthesis made from PET Dacron (registered trade mark). The template acts as a reference for cutting a film from the sheet having dimensions suitable for covering the prosthesis surface.

The prosthesis body and the cut copolymer film are then mounted on respective mandrels of a machine having counter-rotating rollers. The mandrel on which the film is mounted is advantageously formed from an anti-adhesive material and is heated and, during the operation of the machine, is in pressure contact with the other mandrel. Rolling is therefore achieved which, due to the combined action of heat and pressure, causes the copolymer film to adhere to and partly penetrate the body of the prosthesis.

Advantageously, there is an overlap between adjacent edges of film in order to obtain a continuous impermeable coating on the prosthesis body. Greater pressure is exerted in the overlapping zones than in the non-overlapping zones in order to obtain a layer of uniform thickness.

The coated prosthesis obtained in this way is then placed in an oven for approximately one hour at a temperature of 85°C in order to improve the regularity of the coating layer and its adhesion to the prosthesis body.

The characteristics of the coating have been verified using the following tests:

I) Tests for verifying the impermeability of the coated prostheses, carried out with both water and air.
In the first case, complete prostheses (closed on one side) and circular samples of the same material were subjected to the action of a column of water - of such a height as to exert a pressure analogous to physiological pressure (16 kPa) - and any losses measured.
In the second case, a fluid circuit was blocked with discs of the material under examination, pressurised, and any decrease in the pressure caused by leakage of air across the material forming the discs was then measured.

II) Tests after stressing, carried out as in I), but utilising circular samples previously subjected, three times, to separation stresses and laceration of the coating. These stresses were caused by causing the coated side of the samples to adhere under pressure to a smooth polycarbonate surface and then detaching them therefrom by means of a pulling action reproducibly exerted by an elastic means.

Since, in some cases, vascular prostheses made from polyethyleneterephthalate are coated with thin films of turbostatic carbon, known as "Carbofilm", in order to improve their biocompatibility, and since this treatment, by reducing the hydrophobicity of the surface, could reduce the adhesion of the hydrophobic, biodegradable polymer to the prosthesis body, the adhesion of copolymer films to Carbofilm-coated surfaces was also tested.

To this end, the sheets are caused to adhere to these surfaces by exerting pressure and thermal treatment for one hour at 85° C. The polymer of the sheet is then incised to form a network of incisions, following which a strip of adhesive tape is then adhered (under pressure) to this sheet and then detached therefrom by pulling. Whether or not total or partial separation of the copolymer sheet from the Carbofilm-coated surface occurred, and the extent of such separation, provides a good indicator of the adhesion between the sheet and the coated surface.

The tests demonstrated that the copolymer coating provides the prostheses with an impermeability equal to or greater than that of analogous collagen-impregnated prostheses currently in clinical use, that this impermeability remains even after the exertion of forces to separate the copolymer from the textile (or mesh), and that the adhesion between the copolymer sheet and the Carbofilm-coated surface is adequate, in that no separation was observed following the test described.

EXAMPLE 2

This example is similar to the above example with the difference that the polymer sheet of desired thickness made from D,L-lactide and ε-caprolactone copolymers is produced thermo-mechanically. To this end, a suitable quantity of copolymer is inserted in a cavity of desired depth and shape in a mould fitted in a press provided with plates of heated anti-adhesive material. The combined action of heat and pressure causes the excess copolymer to melt and this excess is discharged through apertures formed in the mould. A copolymer sheet of the desired thickness is thus obtained, a portion of which is then applied to a prosthesis body in a manner similar to that described above.

In this case also, the tests described in Example 1 are performed, obtaining results entirely analogous thereto.

EXAMPLE 3

A solution of between 2% and 5% concentration of D,L-lactide copolymer and ε-caprolactone in a solvent such as, for example, acetone, 2-butanone, dimethyleacetamide or mixtures thereof is prepared. An inert propellant is then added to this solution which is sprayed, using known spraying techniques, onto a prosthesis body mounted on a rotating mandrel. The drops striking the surface of this body still contain a good part of the solvent and can therefore coalesce with each other to form a homogenous and continuous layer that does not, however, adhere perfectly to the surface of the prosthesis body, but traces the geometry and adheres partly thereto.

In order to obtain perfect adhesion, the prosthesis coated with this imperfectly adhered layer is mounted on a mandrel of a machine having counter-rotating rollers. During the operation of the machine, this mandrel, which acts as a first roller, comes into pressure contact with a second, counter-rotating roller made of heated anti-adhesive material, to obtain the perfect adhesion of the copolymer layer to the prosthesis body due to the combined action of heat and pressure.

The thickness of the coating layer can be increased by spraying further coats of the copolymer solution.

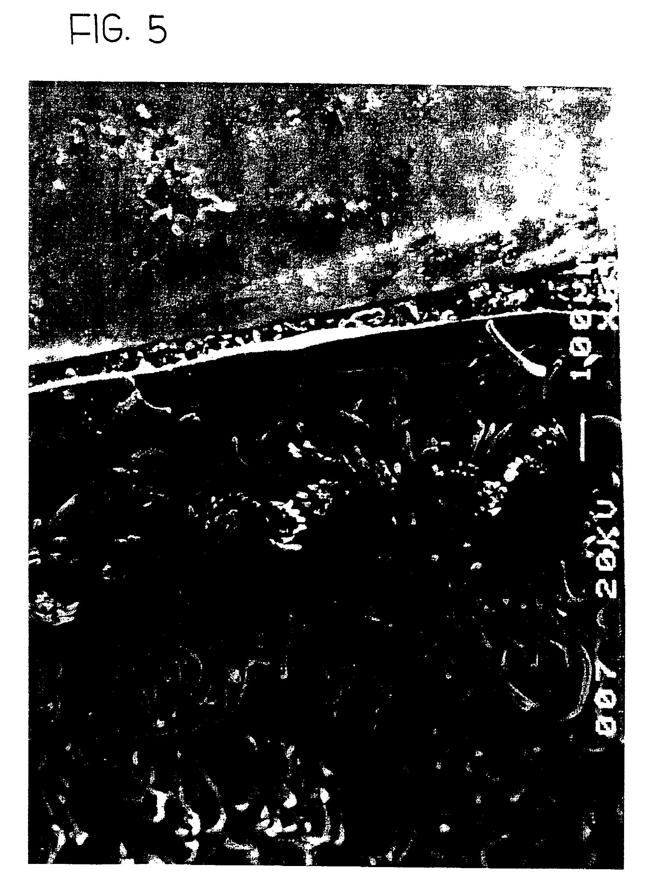

The coated prosthesis thus obtained is then placed in an oven for approximately four hours at a temperature of 55°C in order to improve the regularity and homogeneity of the coating layer (see Figure 5).

Tests, including tests following stressing, were then performed on the coated prosthesis as described in Example 1, obtaining confirmation, in this case also, of its good impermeability.

EXAMPLE 4

This is similar to Example 3 with the differences that a less volatile solvent (or solvent mixture) such as 2-butanone is used, and that the prosthesis body is preliminarily impregnated using the same solvent. By virtue of these measures, the first layer deposited by means of spraying adheres to and partly penetrates the prosthesis body. It is not therefore necessary to perform the hot rolling stage in this case. Instead, one proceeds directly to a thermal stabilisation treatment at 55°C for 90 minutes and, finally, to spraying successive layers until the desired thickness of approximately 150 mm is reached.

Tests, also following stressing - as described in Example 1 - are then performed, which have confirmed, in this case also, its good impermeability.

Naturally, with the principle of the invention remaining the same, the details of construction and the embodiments can be widely varied with respect to that described purely by way of example, without by this departing from the ambit of the invention.

**Claims**

1. A vascular prosthesis having a body made from biostable and biocompatible polymeric material, characterised in that at least a surface portion thereof is coated with at least one impermeable layer based on a synthetic, biodegradable and biocompatible polymer having a coefficient of elasticity less than 10 MPa.

2. A prosthesis according to Claim 1, characterised in that the said polymer is hydrophobic and anhydrous.

3. A prosthesis according to Claim 1 or Claim 2, characterised in that the said polymer is a poly-(lactide-caprolactone) copolymer having an intrinsic viscosity [η], measured in chloroform at 25°C, of less than 4 dl/g.

4. A prosthesis according to Claim 3, characterised in that the said copolymer is obtained from D,L-lactide and ε-caprolactone in a weight ratio of between 40%/60% and 60%/40%.

5. A prosthesis according to Claim 4, characterised in that the said weight ratio is equal to 50%/50%.

6. A prosthesis according to Claim 1 or Claim 2, characterised in that the said polymer is a homopolymer or copolymer of polyvalerolactone, ε-decalactone, polyhydroxybutyrate, polyhydroxyvalerate or polydioxanone.

7. A prosthesis according to Claim 6, characterised in that the said polymer is supplemented by monomers or oligomers of the same polymer, lecithin, polyvinyl pyrrolidone (PVP), esters of citric acid, esters of trimellitic acid and/or glycerololigolactides.

8. A prosthesis according to any preceding claim, characterised in that the said at least one impermeable layer is between 50 and 300 μm thick.

9. A method for the production of a prosthesis according to any preceding claim, characterised in that the said at least one impermeable layer is applied to the said body using coating techniques, in particular, spraying.

10. A method for the production of a prosthesis according to any of Claims from 1 to 8, characterised in that the said at least one impermeable layer is applied to the body using rolling techniques.

11. A method according to Claim 9 or Claim 10, characterised in that the said application is effected at temperatures higher than room temperature.

12. A method according to any of Claims from 9 to 11, characterised in that the said application is followed by a heat treatment.

13. A method according to any of Claims from 9 to 12, characterised in that it provides for the combination or successive utilisation of coating and rolling techniques for applying the said at least one impermeable layer.

14. A method according to any of Claims from 9 to 13, characterised in that it provides for the utilisation of various techniques for the application of the at least one impermeable layer on areas of a single prosthesis having different geometries.

# FIG. 1

CL-DLLA COPOLYMERS: GLASS TRANSITION AND MELTING TEMPERATURES

| %PCL | Tg | Tm | %PDLLA |
|------|------|------|--------|
| 100 | -60 | 59.7 | 0 |
| 80 | -50.9 | 36.5 | 20 |
| 60 | -28 | | 40 |
| 40 | -1 | | 60 |
| 0 | 49 | | 100 |

# FIG. 2

# FIG. 3

| % CL | % DLLA | COEFFICIENT OF ELASTICITY (MPa) | MAXIMUM LOAD (KPa) | BREAKING STRAIN (KPa) |
|------|--------|--------------------------------|--------------------|------------------------|
| 100 | 0 | 260 | 5000 | 15000 |
| 80 | 20 | 34 | 1400 | 1300 |
| 60 | 40 | 6,4 | 13 | 6 |
| 40 | 60 | 3 | 70 | 10 |
| 0 | 100 | 1540 | | |

# FIG. 4

| % DLLA | % CL | Kα | 1Kα(DAYS) |
|--------|------|---------|-----------|
| 0 | 100 | 2,46E-03 | 406,5 |
| 11 | 89 | 1,54E-02 | 64,9 |
| 27 | 73 | 5,07E-02 | 19,7 |
| 100 | 0 | 6,31E-03 | 158,5 |

FIG. 5